# EUROPEAN PATENT APPLICATION

(11) **EP 0 842 937 A2**
(43) Date of publication of application: **20.05.1998**
(21) Application number: 97120169.4
(22) Date of filing: 18.11.1997
(51) Int. Cl.: C07D 501/06, C07D 501/34, C07D 501/36

(54) **Process for the preparation of the cephalosporin derivatives cefotaxime and ceftriaxone**

(30) Priority: 19.11.1996 IT MI962406
(71) Applicant: Hichem Pharma S.p.A., 20121 Milano (IT); S.B.D. S.r.l., 20121 Milano (IT)
(72) Inventor: Monguzzi, Riccardo, 20079 Marudo (Lodi) (IT); Menapace, Silvano, 20098, Sesto Ulteriano (Milano) (IT); Anzaghi, Piergiorgio, 20098, Sesto Ulteriano (Milano) (IT)
(74) Representative: Minoja, Fabrizio

(57) **Abstract**

A process for the preparation of the cephalosporanic antibiotics CEFOTAXIME and CEFTRIAXONE.

Said process consists in reacting 7-aminocephalosporanic acid (7-ACA) or 7-amino-3[(2,5-dihydro-6-hydroxy-2-methyl-5-oxo-as-triazin-3-yl]-3-cephem-4-carboxylic acid (7-ACT) with suitably activated 4-chloro-2-methoxyimino-3-oxobutyric acid, and subsequently cyclizing the intermediate chloromethoxyimino oxobutyramide with thiourea.

## Description

The present invention relates to a method for the preparation of cephalosporanic acid derivatives, in particular for the preparation of the compounds known under the names CEFOTAXIME and CEFTRIAXONE.

CEFOTAXIME or 7-β-2-[(2-amino-4-thiazolyl)-2-methoxyiminoacetamido]cephalosporanic acid, and CEFTRIAXONE or 7-β-[2-(2-amino-4-thiazolyl)-2-methoxyiminoacetamido]-3-[[(2,5-dihydro-6-hydroxy-2-methyl-5-oxo-as-triazin-3-yl)thio]methyl]-3-cephem-4-carboxylic acid, corresponding to formula (I) 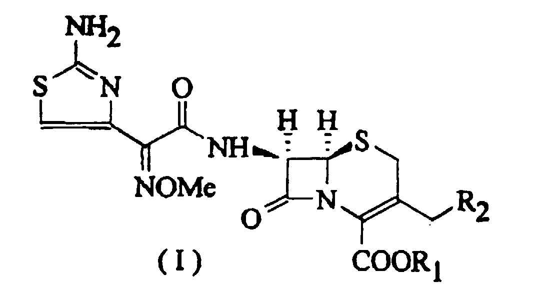 R₂ = OCOMe; 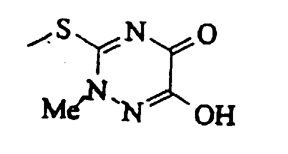 R₁ = H; Na; Benzathine
wherein R¹ is a hydrogen atom or a salt with an alkali metal or with an organic base, and R² is an acetoxy group or a (2,5-dihydro-2-methyl-6-hydroxy-5-oxo-as-triazin-3-yl)thio group, are two antibiotics widely used thanks to their effective, wide-spectrum antimicrobial activity.

Up to now, a number of processes for the preparation of such two semi-synthetic cephalosporins have been described. For example, it is known that the compounds of formula (I) can be synthesized by conversion of the organic acid of formula (A) 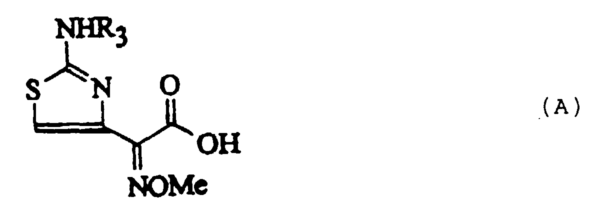 wherein R³ is an amine-protecting group such as trityl, chloroacetyl, formyl, into a carboxyl-activated derivative, and subsequent acylation with 7-ACA or 7-ACT.

Acid chlorides, anhydrides, esters, amides, amides in the solvated form and the like can be cited as carboxylic group activators.

In order to use the acid chlorides as activated derivatives, the organic acid (A) can be reacted with thionyl chloride (SOCl₂). The resulting acid chloride is used for the acylation of 7-ACA and of the derivatives thereof. The subsequent removal of the protective group R³ yields compounds of formula (I): see Japanese Patent Applications Kokai N. 52-102,096; 53-34,795; 54-68,1976; 54-52,096 and 54-157,596; British Patent n. 2,025,933.

The factors limiting the applicability of the processes mentioned above are the unfavourable operative conditions in which said reactions have to be carried out (low temperatures, absolute dryness etc.), the protection and subsequent deprotection of the amino group, the difficulty in recovering the chloride due to its instability, and the like.

Other methods comprise the preparation of specific active esters starting from the acid of formula (B) 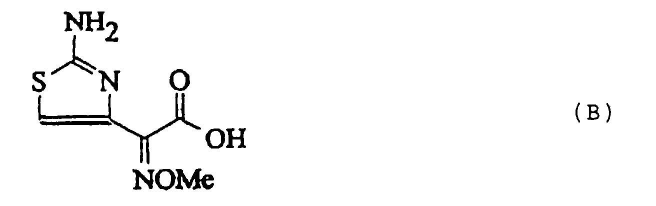 with 1-hydroxybenzothiazole and dicyclohexylcarbodiimide and subsequent acylation of the ester with 7-ACA and 7-ACT, as reported in Japanese Patent Applications Kokai N. 52-102,293 54-95,593 and 56-152,488.

This process provides low yields due to side-reactions occurring during the preparation of the activated esters.

In specific conditions (European Patent n. 175,814) the reaction mentioned above leads to an active amide in the solvate form which can be used for acylating 7-ACA and 7-ACT, to give compounds of formula (I) in good yields.

The process has however drawbacks in that the solvate is difficult to recover and the reaction times are long.

Further activations of the compound of formula (B) precede the in situ formation of acyloxyphosphonium chloride of formula 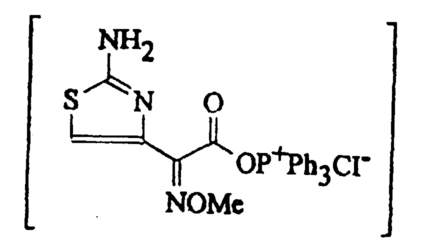 as reported in WO 93/10123 and the formation of thioesters with pyridine and benzothiazole followed by the acylation with 7-ACA and derivatives (see Japanese Patent Applications Kokai n. 52-102,293; 54-95,593 and 56-152,488).

### DISCLOSURE OF THE INVENTION

The process of the present invention is based on the condensation of suitably activated 4-chloro-2-methoxyimino-3-oxobutyric acid with 7-β-aminocephalosporanic acid (7-ACA) or with a derivative thereof, suitably substituted at the 3- position (7-ACT). The resulting compound is not recovered, but is directly condensed with thiourea in hydroalcoholic medium to obtain the desired cephalosporanic acid derivatives.

The synthesis starts from tert-butyl 2-acetyl-2-hydroxyimino acetate (I) which can be obtained as described by H. Yamanaka et al. in J. of Antibiotics 1738 (1985), and which is transformed into the corresponding methoxyimino derivative (II) in the presence of (CH₃)₂SO₄ and K₂CO₃.

The 2-acetyl-2-methoxyimino acetic acid tert-butyl ester (II) is then transformed into the corresponding chloro derivative, at the same time removing the tert-butyl protective group by reaction with sulfuryl chloride; 4-chloro-2-methoxyimino-3-oxobutyric acid (III) is thereby obtained, which is in its turn activated as mercaptobenzothiazolyl ester (V) by means of dithiobisbenzothiazole (IV) and triphenylphosphine in the presence of triethylamine and/or ethyldiisopropylamine.

The 4-chloro-2-methoxyimino-3-oxobutyric acid activated ester (V) can be condensed with the desired cephalosporanic substrate (VI) to obtain, depending on the use of either 7-ACA or of 7-ACT, an intermediate compound which is not recovered, but is directly condensed with thiourea in hydroalcoholic medium to give the desired cephalosporins (I).

The condensation of the activated ester takes peace in water - tetrahydrofuran medium at a low temperature, in the presence of triethylamine and ethyldiisopropylamine. The mercaptobensothiazole formed during the reaction can be removed with organic washings.

The subsequent condensation with thiourea in hydroalcoholic medium, leads the geometry of the methoxyimine present on the amido chain to the desired sense.

The resulting compounds can be precipitated as acid solvates, (this is preferred in the case of CEFOTAXIME which, still humid, is transformed into the acetone solvate by mild heating with acetone) or as N,N'-dibenzilethylenediamine salt (as takes place for CEFTRIAXONE).

The process can be schematized as follows: 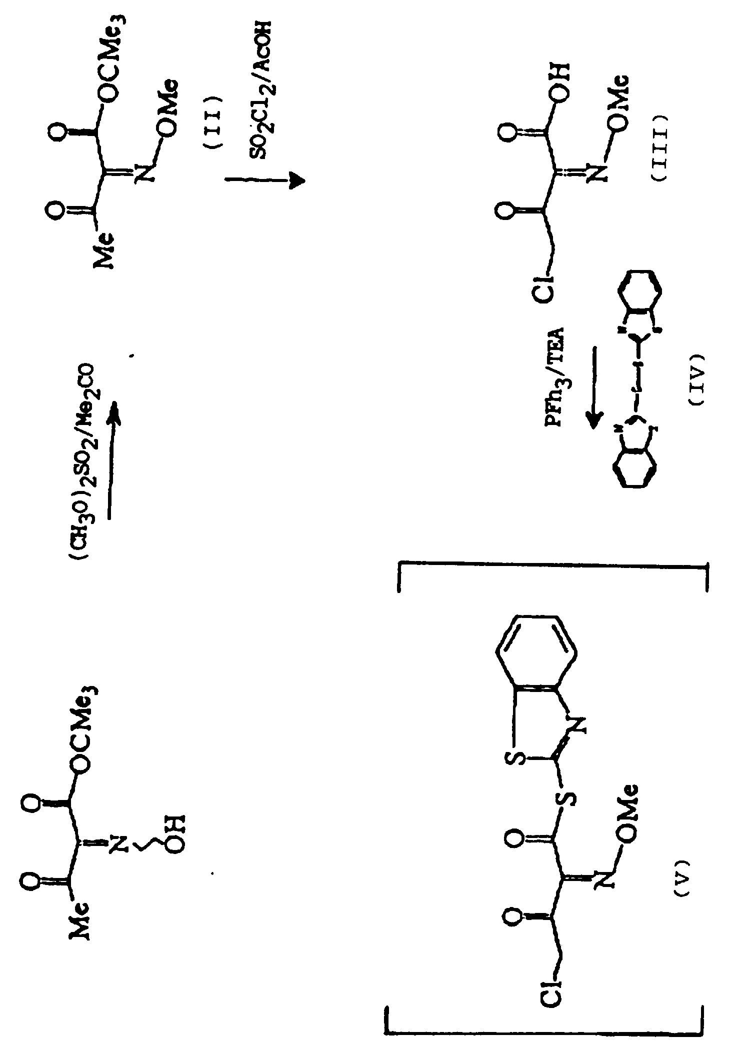 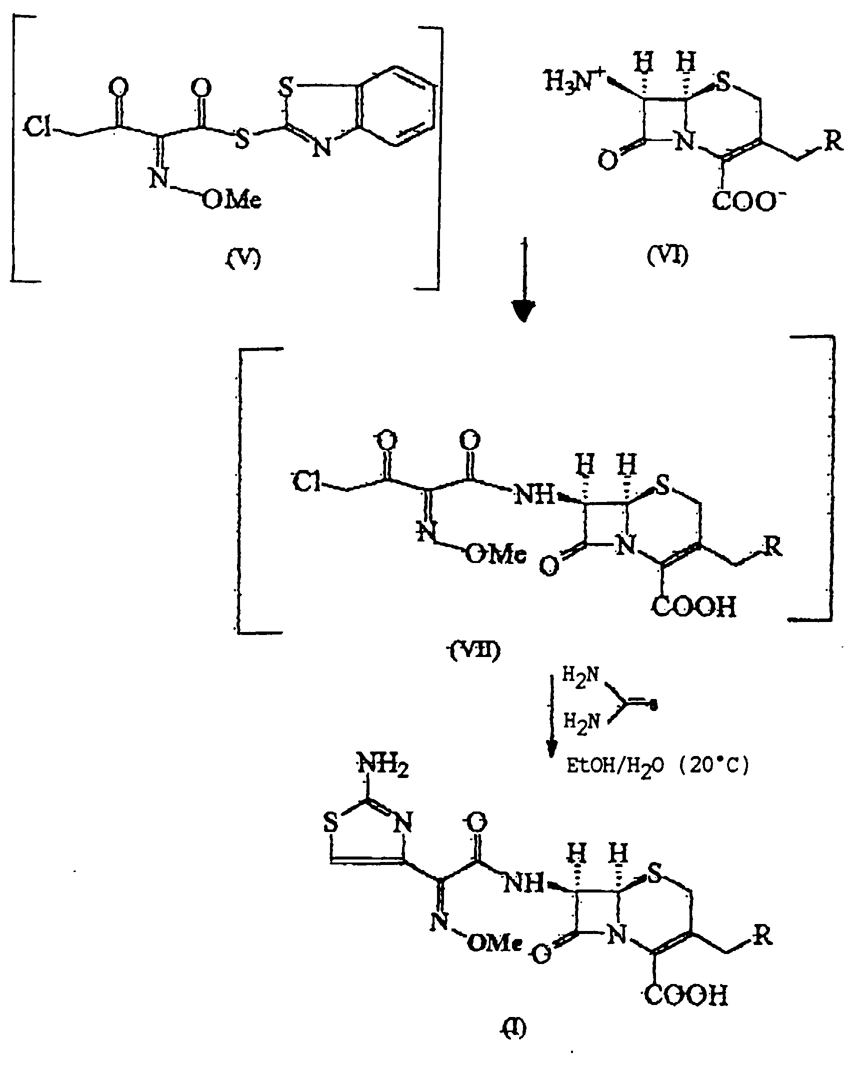 R² = OCOMe; 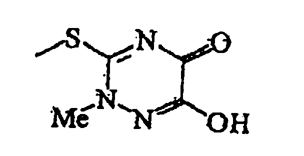 R¹ = H; Na; benzathine

Compound (I) having R² = (2,5-dihydro-2-methyl-6-hydroxy-5-oxo-as-triazin-3-yl)thio, and R¹ = Na or benzathine, can be conveniently prepared from compound (I), in which R² = OCOCH³ and R¹ = H, as the acetone solvate, by silylation with O,N-bis-trimethylsilylacetamide (BSA) in acetonitrile, then treatment with trimethyliodosilane (Me₃SiI) prepared in situ from iodine and hexamethyldisilane. In these conditions the acetoxy group is transformed into the iodomethyl derivative, which is not recovered due to its instability, but is reacted with 3-mercapto-2-methyl(2,5-dihydro-6-hydroxy-2-methyl-5-oxo)-as-triazine also previously silylated with BSA in acetonitrile/dimethylacetamide, to give the compound of formula (I) wherein R² is thiotriazinone and R¹ has the meanings described above, according to the scheme reported hereinbelow:

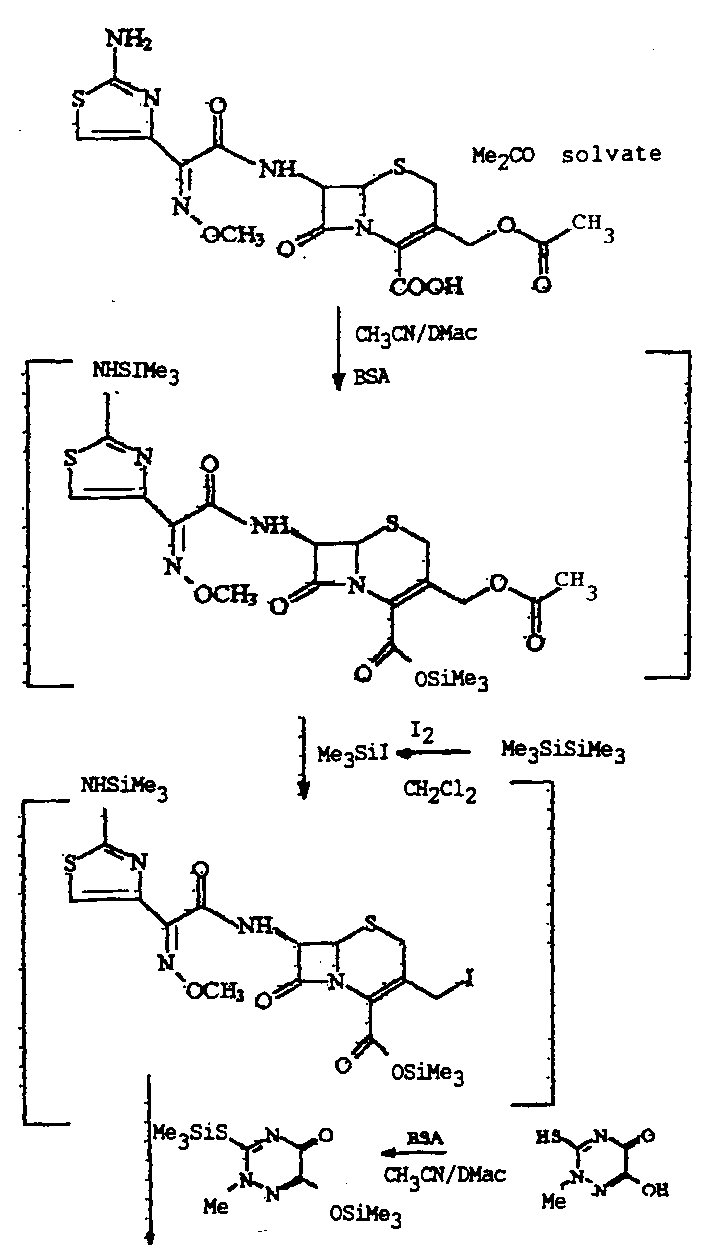

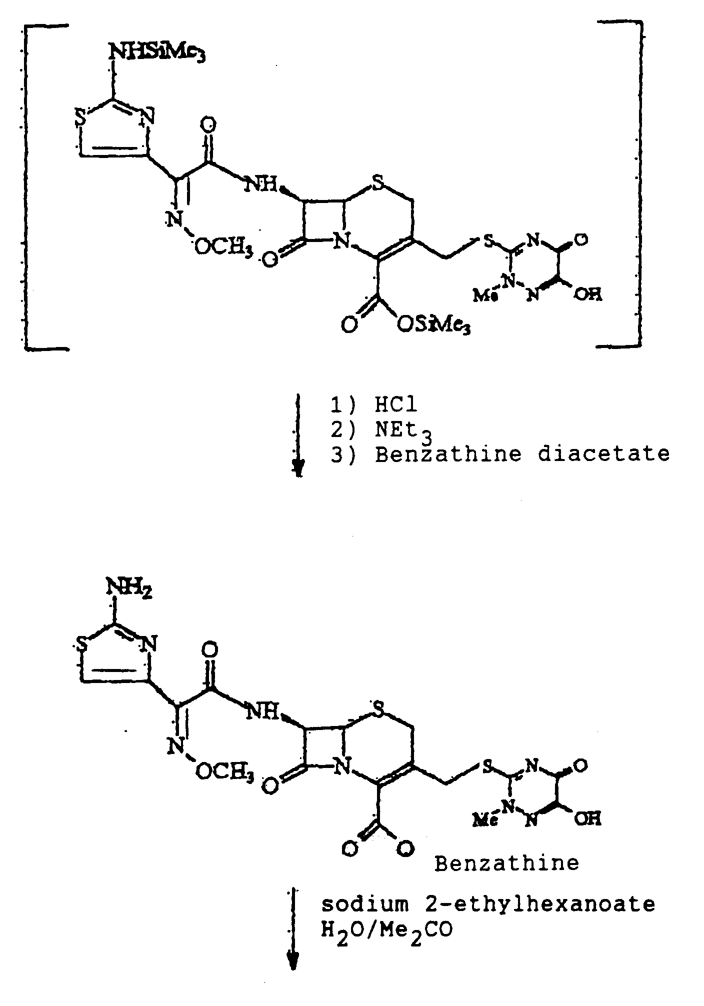

The following Examples further illustrate the invention.

### Example 1 - Tert-butyl 2-acetyl-2-methoxyiminoacetate (II)

215 g of tert-butyl 2-acetyl-2-hydroxyimino acetate (prepared according to the method by H. Yamanaka et al., J. of Antibiotic 1738 (1985)) are added to 1000 ml of acetone. The mixture is cooled to 15°C and added with 235 g of K₂CO₃, then with 105 ml of (CH₃)₂SO₄ (dimethyl sulfate). The mixture is stirred for four hours at room temperature, then poured into 4 l of water and repeatedly extracted with methylene chloride.

The organic phase is washed with water, dried and evaporated under vacuum, thereby obtaining 220 g of the desired compound.
¹H-HMR: (DMSO δ6) 1.41 (9H, s), 2.21 (3H, s), 3.90 (3H, s).

### Example 2 - 4-Chloro-2-methoxyimino-3-oxobutyric acid (III)

150 g of sulforyl chloride are dropped into a solution of 50 g of the compound of Example 1 in 50 ml of acetic acid at 50° in 3.5 hours. The reaction mixture is cooled at room temperature, left to stand for one hour, then concentrated under vacuum. The resulting residue is redissolved in 250 ml of ethyl acetate and this solution is washed with brine. The organic phase is dried, then evaporated under vacuum.
Yield = 60%.
¹H-HMR DMSO 3.90 (3H, s), 4.75 (2H, s), 10.8 (1H, s all).

### Example 3 - Cefotaxime acetone solvate

### a) Preparation of thioester (V)

500 ml of THF are added with 75 g of the compound of Example 2 in the presence of 26.6 g of triethylamine. After that, 167 g of dithio-bis-benzothiazole are added at room temperature, then 132 g of triphenylphosphine are added, the reaction being slightly exothermic. The mixture is stirred for one hour until HPLC evidences the disappearance of the chloroacid.

### b) Preparation of cefotaxime acetone solvate

The mixture is cooled to 0°C and left at this temperature for one hour, then filtered. The filtrate is added with a solution of 50 g of 7-ACA salified as TEA salt (18.5 g) in 500 ml of water, cooled at 0-5°C, keeping this temperature until HPLC shows the disappearance of 7-ACA. 500 ml of ethyl acetate are added, keeping the mixture under stirring for 10'. The organic phase containing mercaptobenzothiazole is discarded. The stirred aqueous phase is added with 500 ml of ethanol, then with 27 g of thiourea and 38 g of sodium acetate, keeping stirring for 16 hours. The mixture is then poured into 500 ml of ethyl acetate, with stirring. The organic phase is discarded and the aqueous solution is poured in 150 ml of 99% formic acid. The mixture is kept at room temperature for 1 hour, the resulting precipitate of CEFOTAXIME in the form of formic acid solvate is filtered, then transformed into the acetone solvate without drying, by mild heating with acetone at 40°C.
68 g of compound are obtained.

The intermediate thioester of Example 3 was recovered as a pale yellow solid by silica gel chromatography.
M.p. 145-47°C (dec); TLC: R_{f} 0.81 (CH₂Cl₂); R_{f} 0.15 (Hexane-methylene chloride; 7:3)
¹H-NMR DMSO-CDCl₃ (1:1 V/V) δ: 4.07 (3H, s); 4.75 (2H, s); 7.4-7.5 (3H, m); 8.40 (1H, dd; J=6; 2Hz)
CMR (DMSO) δ_{c}: 193.2; 185.2; 160.0; 147.8; 138.2; 127.9; 127.1; 126.8; 122.9; 116.4; 64.8; 45.6.

### Example 4 - CEFTRIAXONE benzathine salt

500 ml of water kept at 0-5°C is added with 68 g of 7-β-amino-3-[[(2,5-dihydro-6-hydroxy-2-methyl-5-oxo-1,2,4-triazin-3-yl)thio]methyl]-3-cephem-4-carboxylic acid (7-ACT). After that, the reaction mixture of the thioester (V) from Example 3 is added, keeping this temperature, until HPLC evidences the disappearance of ACT. 500 ml of ethyl acetate are added, stirring the mixture for 10′. The organic phase containing mercaptobenzothiazole is discarded. The stirred aqueous phase is added with 500 ml of ethanol, 27 g of thiourea and 38 g of sodium acetate. The mixture is stirred for 16 hours, then poured into 500 ml of ethyl acetate, keeping stirring for 15′. The phases are partitioned and the organic phase is discarded. The aqueous phase which contains the compound as TEA salt is added with 70 g of N,N'-dibenzylethylenediamine diacetate under stirring, to complete the precipitation of CEFTRIAXONE benzathine salt (g 140).

CEFTRIAXONE sodium salt can be obtained from the benzathine salt, as disclosed in U.S. Pat. n. 5,026,843.

### Example 5 - CEFTRIAXONE benzathine salt via iodomethyl derivative

A 1000 ml round-bottomed flask (A) in anhydrous conditions and under nitrogen atmosphere is loaded with 270 ml of acetonitrile and 25,4 g of iodine (100 mmoles), then temperature is brought to 30°C and 24,3 ml of hexamethyldisilane (123 mmoles) are dropped therein. The reaction mixture is warmed to 40°C and kept at this temperature for 16 hours, after which it is cooled to 0°C.

Separately, a 250 ml round-bottomed flask (B) in anhydrous conditions and under nitrogen atmosphere is loaded with 40 ml of dimethylacetamide, 40 ml of acetonitrile and 18.8 g (36,7 mmoles) of CEFOTAXIME acetone solvate, then 18.4 ml (75 mmoles) of 0,N-bis-trimethylsilylacetamide are dropped into the mixture, with stirring, until complete dissolution. The reaction mixture is cooled at 0°C in 30′, then poured into the trimethylsilyl iodide solution prepared in situ in the above flask A). At the end of the addition, the temperature is raised to 10°C and the mixture is stirred until the reaction is completed.

A 250 ml round-bottomed flask (C) under nitrogen atmosphere is loaded with 6.62 g (41.6 mmoles) of 1,2,5,6-tetrahydro-2-methyl-5,6dioxo-1,2,4-triazin-3-yl-thiol and 53.8 (264.4 mmoles) of O,N-bis-trimethylsilylacetamide, keeping stirring for 4 hours and the resulting suspension is cooled to 5°C, then poured into flask (A) containing the reaction mixture prepared above, with stirring, in 15′ and under nitrogen atmosphere.

When the reaction is completed, 20 ml of 5% HCl are added, stirring for 15′. Temperature is allowed to reach +15°C, then 250 ml of water and 250 ml of ethyl acetate are added. Keeping a temperature of +15°C, triethylamine is dropped until pH 6. The mixture is stirred for 15 minutes, the organic phase is separated; the aqueous phase is washed twice with 100 ml of ethyl acetate, then it is stirred and a solution of 13.23 g (36.7 mmoles) of dibenzylethylenediamine in 53 ml of water is dropped therein. The mixture is stirred for 1 hour at +15°C, then filtered and washed with water (4 x 20 ml). The humid precipitate is suspended in a mixture of 50 ml of a solution of 9.85 g (59.1 mmoles) of sodium 2-ethylhexanoate in 100 ml of ethyl acetate, stirring for 1 hour at room temperature, then filtered again and washed with acetone (4 x 20 ml).

15.15 g of CEFTRIAXONE disodium salt are obtained.

## Claims

1. A process for the preparation of the compounds of formula (I) and the pharmaceutically acceptable salts thereof, 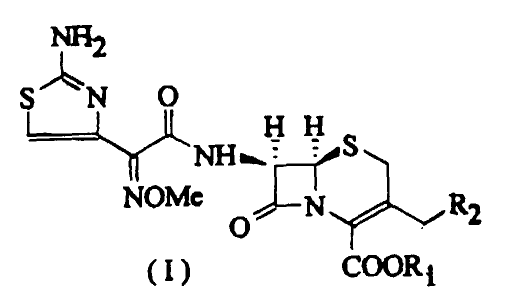 R₂ = OCOMe; 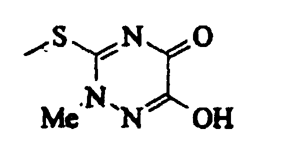 R₁ = H; Na; Benzathine;
which process comprises the following steps:
a) reacting a 4-chloro-2-methoxyimino-3-oxobutyric acid activated ester (V) (non isolated) with a compound of formula (VI); 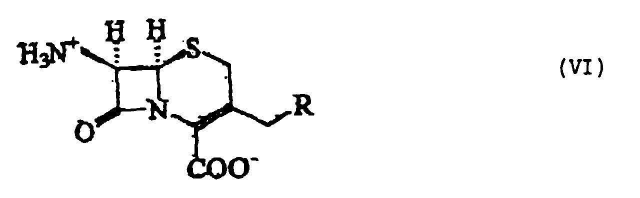 wherein R has the meanings defined above for R², or is a group which can be transformed into R²;
b) reacting the compound from step a), without recovering it, with thiourea in a hydroalcoholic medium to give (I).

2. A process according to claim 1, wherein compound of formula (I) is CEFTRIAXONE.

3. A process according to claim 1, wherein compound of formula (I) is CEFOTAXIME.

4. A process according to claim 1, wherein the activated ester (V) of step a) is the 2-mercaptobenzothiazolyl ester.

5. A process according to claim 4, wherein 2-mercaptobenzothiazolyl ester is obtained according to the following scheme:

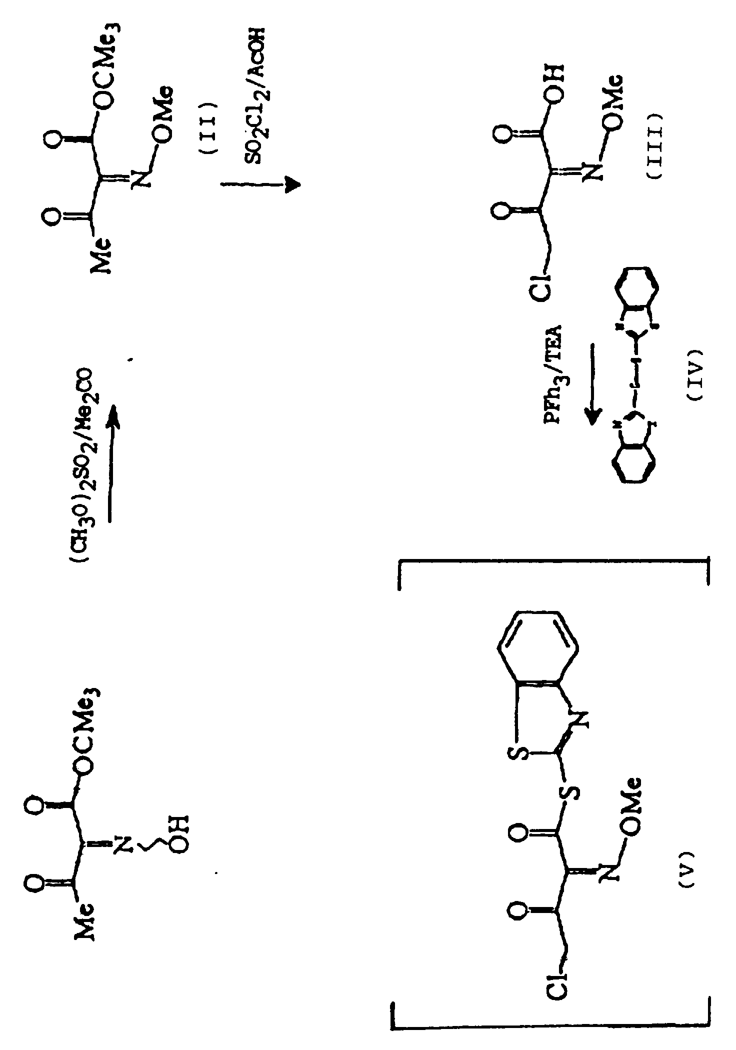

6. A process for the preparation of CEFTRIAXONE from CEFOTAXIME, which process comprises the following steps:
a) silylating CEFOTAXIME acetone solvate with O,N-bis-trimethylsilylacetamide;
b) treating the compound from step a) with trimethyliodosilane prepared in situ from iodine and hexamethylsilane in acetonitrile, to give the 3-iodomethyl derivative;
c) reacting the 3-iodomethyl derivative from step b) with trimethylsilylated 3-mercapto-2-methyl(2,5-dihydro-6-hydroxy-2-methyl-5-oxo)-as-triazine;
d) treating the compound from step c) in succession, with acids and bases until obtaining the desired CEFTRIAXONE salt.

7. A process according to claims 1 - 5, wherein the activate ester (V) is not isolated.

8. A process according to claims 1, 4, 5, 6 for the preparation of CEFTRIAXONE benzathine salt.

9. A process according to claim 1 for the preparation of CEFOTAXIME benzathine salt.
